# EUROPEAN PATENT APPLICATION

(11) **EP 4 841 649 A1**
(43) Date of publication of application: **18.11.2026**
(21) Application number: 25176774.5
(22) Date of filing: 15.05.2025
(51) Int. Cl.: G16H 50/50, G09B 23/28, G16H 50/70

(54) **SIMULATING MEDICAL IMAGING SYSTEM**

(71) Applicant: Koninklijke Philips N.V., 5656 AE Eindhoven (NL)
(72) Inventor: GRAESSLIN, Ingmar, Eindhoven (NL); DONEVA, Mariya Ivanova, Eindhoven (NL); AMTHOR, Thomas Erik, 5656AG Eindhoven (NL)
(74) Representative: Philips Intellectual Property & Standards

(57) **Abstract**

Proposed concepts thus aim to provide schemes, solutions, concepts, designs, methods and systems pertaining to simulating an output of a medical imaging system. In particular, embodiments aim to provide a method for simulating an output of a medical imaging system by simulating how a medical imaging system would image an anatomical model generated using patient information describing features of an example patient.

## Description

### FIELD OF THE INVENTION

This invention relates to the field of simulating an output of a medical imaging system.

### BACKGROUND OF THE INVENTION

Magnetic Resonance Imaging (MRI) is essential for many accurate medical diagnoses, requiring radiology staff to undergo specialized training to operate the machines effectively. Traditional training methods involve using actual MRI scanners which can disrupt daily medical operations and reduce the availability of machines for patient care. Moreover, training on real scanners may expose trainees to sensitive patient information, raising privacy concerns. The same also applies to many other medical imaging systems such as CT, X-ray, ultrasound, etc.

There is therefore need for a training solution which minimizes these disruptions, and ideally protects patient data and provides personalized learning experiences for medical staff.

### SUMMARY OF THE INVENTION

The invention is defined by the claims.

According to examples in accordance with an aspect of the invention, there is provided a computer-implemented method for simulating an output of a medical imaging system.

The method comprises: generating an anatomical model of a simulated patient based on patient information describing physiological features of an example patient; generating a synthetic medical image of a simulated medical imaging system based on the anatomical model; and generating a display control signal describing the synthetic medical image.

Proposed concepts thus aim to provide schemes, solutions, concepts, designs, methods and systems pertaining to simulating an output of a medical imaging system. In particular, embodiments aim to provide a method for simulating an output of a medical imaging system by simulating how a medical imaging system would image an anatomical model generated using patient information describing features of an example patient.

The inventors have realised that patient information describing physiological features of an example patient (e.g., using the data of one or more real patients) can be used to generate an anatomical model of a simulated patient. How a medical imaging system would image said anatomical model can then be simulated to generate a synthetic medical image which can be displayed/output to the user, e.g., for training purposes.

Furthermore, generating an anatomical model and simulating a medical imaging system allows for flexibility, for example, a user can practice using a medical imaging system and adjust imaging settings or move the anatomical model inside the simulated medical imaging system to observe how this affects the synthetic medical image. In this way, a remote training system is offered which can, for example, utilize MRI simulators and virtual participation to enable training without interfering with clinical workflows or requiring access to physical MRI machines.

Ultimately, an improved method for simulating an output of a medical imaging system is provided.

In some embodiments, the method may further comprise receiving a control signal comprising a control parameter of the simulated medical imaging system, wherein the synthetic medical image is generated further based on the control signal. This allows the simulation to be adjusted in some way thereby enhancing the customizability of the simulation.

In some embodiments, the control parameter may comprise a selection of anatomy of the anatomical model. This facilitates the anatomy to-be-imaged being selected, thereby enhancing the customizability of the simulation.

In some embodiments, the control parameter may comprise at least one imaging setting of the simulated medical imaging system. This allows the imaging settings of the simulated medical imaging system to be adjusted, and therefore how imaging settings affect the output medical images can be simulated and studied.

In some embodiments, the control signal may further comprise positioning information indicating a first position of the simulated patient in the simulated medical imaging system. This allows the simulated patient's position in the simulated medical imaging system to be adjusted, and therefore how a patient's position affects the output medical images can be simulated and studied.

In some embodiments, generating the synthetic medical image may comprise using a Bloch simulation algorithm. This has been found to be a particularly useful simulation algorithm for helping to generate the synthetic medical image.

In some embodiments, the patient information may comprise a patient-specific feature. This allows a user to see what medical images of patients with unique or rare features look like. In this way, their learning may be enhanced.

In some embodiments, the patient-specific feature may comprise at least one of: an implant; a bone fracture; a soft tissue injury; a tumor; a surgical modification; scar tissue; inflammation; an anatomical abnormality; and a foreign body. These are all useful types of features for medical images thereof to be simulated, thus allowing a user to learn how a variety of different features manifest in a medical image.

In some embodiments, the method may further comprise generating a patient behavioral model describing a position and movement of the simulated patient in the simulated medical imaging system, wherein the synthetic medical image is generated further based on the patient behavioral model. This allows the simulation to be made more realistic, including patient movement, and may therefore facilitate a user interacting with a patient's movement.

In some embodiments, the anatomical model may comprise a 3D tissue map. This is a particularly useful form for the anatomical model.

In some embodiments, the display control signal may further describe a representation of the simulated medical imaging system. This facilitates a user seeing the simulated medical imaging system in addition to its simulated output.

In some embodiments, the method may further comprise outputting the display control signal to a virtual or augmented reality headset. This allows a user to be provided with the display in an ergonomic and particularly engaging way.

In some embodiments, the control signal may be received by the virtual or augmented reality headset. This allows a user to directly interact with the display, thus providing a more intuitive and lifelike experience.

In some embodiments, the medical imaging system may comprise one of: an MRI system; a CT system; an X-ray system; and an ultrasound system. These are all useful medical imaging systems to simulate the output of.

According to another aspect of the invention, there is provided a computer program comprising code means for implementing the method of any herein disclosed method when said program is run on a processing system.

According to another aspect of the invention, there is provided a system for simulating an output of a medical imaging system. The system comprising a processing arrangement configured to: generate an anatomical model of a simulated patient based on patient information describing physiological features of an example patient; generate a synthetic medical image of a simulated medical imaging system based on the anatomical model; and generate a display control signal describing the synthetic medical image.

Thus, there may be proposed concepts for simulating an output of a medical imaging system, and this may be done based on simulating how a medical imaging system would image an anatomical model generated using patient information describing features of an example patient.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for simulating an output of a medical imaging system according to a proposed embodiment;
Fig. 2 is a flow diagram of a method for simulating an output of a medical imaging system including receiving a control signal according to a proposed embodiment;
Fig. 3 is a flow diagram of a method for simulating an output of a medical imaging system including generating a patient behavioral model according to a proposed embodiment;
Fig. 4 is a simplified block diagram of a system for simulating an output of a medical imaging system according to a proposed embodiment; and
Fig. 5 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to simulating an output of a medical imaging system. According to proposed concepts, a number of possible solutions may be implemented separately or jointly. That is, although these possible solutions may be described below separately, two or more of these possible solutions may be implemented in one combination or another.

Embodiments of the invention aim to provide a method for simulating an output of a medical imaging system. This can be achieved by simulating how a medical imaging system would image an anatomical model generated using patient information describing features of an example patient. The inventors have realised that patient information describing physiological features of an example patient (e.g., using the data of one or more real patients) can be used to generate an anatomical model of a simulated patient. How a medical imaging system would image said anatomical model can then be simulated to generate a synthetic medical image which can be displayed/output.

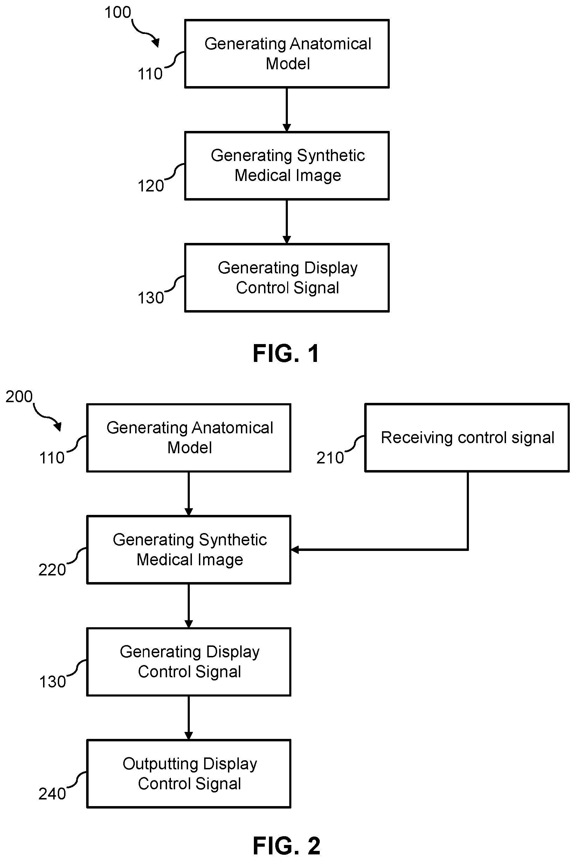

Referring now to Fig. 1, there is depicted a simplified flow diagram of a computer-implemented method 100 for simulating an output of a medical imaging system according to a proposed embodiment.

The method 100 begins with step 110 of generating an anatomical model of a simulated patient based on (i.e., responsive to) patient information. The patient information describes physiological features of an example patient. In other words, the patient information describes specific physiological (biological) characteristics that are representative of a particular example patient (e.g., a real-life patient wherein the patient information is historical information, or a synthetic example patient where the patient information is synthetic information (generated, for example, using a machine-learning model or neural network), or a composite patient wherein the patient information is a combination of historical information from a plurality of real-life patients (i.e., the example patient comprises physiological features of multiple historical patients)). Based on this information, an anatomical model (of a simulated patient) can thus be generated. An anatomical model should be understood as a model or representation of the simulated (i.e., example) patient's anatomy, such as the structure, shape or spatial arrangement of anatomical features (e.g., organs or tissues or bones, etc.). In this embodiment, the anatomical model comprises a 3D tissue map (a 3D tissue map being a volumetric representation of the spatial distribution, type and organization of biological tissues) though in other embodiments, the anatomical model can comprise any other suitable form for representing the simulated patient's anatomy.

In this embodiment, the patient information comprises a patient-specific feature. That is, the patient information comprises a physiological feature which is relatively unique to the example patient, i.e., is a relatively rare physiological feature, i.e., is not present in a standard/default/typical human body. This allows a user to see what medical images of patients with unique or rare features look like, and in this way, their learning can be enhanced. For example, in this embodiment, the patient-specific feature comprises at least one of: an implant; a bone fracture; a soft tissue injury; a tumor; a surgical modification (e.g., a limb amputation, mastectomy, gastric bypass, spinal function, and/or joint replacement); scar tissue; inflammation; an anatomical abnormality (e.g., scoliosis, cleft palate, congenital heart defect, polydactyly, and/or spina bifida); and a foreign body (e.g., an orthopedic implant, a pacemaker, a cochlear implant, and/or ingested objects). These are all useful types of features for medical images thereof to be simulated, thus allowing a user to learn how a variety of different features manifest in a medical image.

Step 120 comprises generating a synthetic medical image of a simulated medical imaging system based on (i.e., responsive to) the anatomical model. In other words, at least part of the anatomical model is input into a system which models how a medical imaging system would image the at least part of the input anatomical model to thus produce a synthetic medical image (of said at least part of the input anatomical model). In this embodiment, the simulated medical imaging system is specifically a simulated MRI system though in other embodiments, the medical imaging system can comprise a CT system, an X-ray system or an ultrasound system. It should also be noted that within each type of medical imaging system, various different models can be emulated as well as their respective user interfaces. This can facilitate users becoming familiar with different systems which they may encounter in real-world settings and a user may be able to select which specific model they would like to simulate.

As the skilled person would be aware, the methodology to generate the synthetic medical image based on the anatomical model can take many forms, for example, a simulation algorithm, a machine-learning algorithm, a neural network, selection from a database of historical medical images (e.g., of a real-life patient which the simulated patient is based on, or of a closest match), etc. In this embodiment, however, generating the synthetic medical image comprises using a Bloch simulation algorithm. As the skilled person would be aware, a Bloch simulation algorithm models how the magnetization of nuclear spins evolves over time under magnetic fields and relaxation effects. It divides time into small steps and updates the magnetization vector at each step based on the applied magnetic field and how the spins return to equilibrium. It typically uses numerical integration methods and may include transformations to a rotating frame to simplify the effects of RF pulses. The result is a time sequence of magnetization states that can be used to simulate MR signals and thus simulate how a magnetic resonance imaging machine would image a given anatomical model. To be clear, a Bloch simulation algorithm is used to help generate the synthetic medical image only when an MRI system is being simulated as it would not be suitable for simulating a CT system, an X-ray system or an ultrasound imaging system. For simulating a CT system, any suitable algorithm may be utilized, for example, Monte Carlo-based photon transport simulation. For simulating an X-ray system, again, any suitable algorithm may be utilized, for example, a ray-tracing model, e.g., with attenuation and scatter modeling. For simulating an ultrasound imaging system, any suitable algorithm may be utilized, for example, a k-Wave-based acoustic propagation model.

Step 130 comprises generating a display control signal describing the synthetic medical image. In other words, a display control signal which comprises a representation of the synthetic medical image generated in step 120 is generated. The generated display control signal may then be subsequently output to a display device or first combined with one or more display control signals or input into another system for analysis, etc.

In this embodiment, the display control signal further describes a representation of the simulated medical imaging system (i.e., in this embodiment, the simulated MRI system). This may allow a user to see the simulated medical imaging system in addition to its simulated output though, of course, this feature is not essential to the functioning of the invention.

In a specific example of the invention, a user selects an example patient to be simulated and have an anatomical model generated of. A user also selects a medical imaging system to simulate (e.g., an X-ray system). A synthetic medical image is then generated showing how the X-ray system would image the simulated patient (e.g., using default settings) and a display control signal is generated describing this synthetic medical image which can then be output to, for example, a display screen of the user. In this way, a user can more quickly and easily learn how a medical imaging system images different patients and how the patients' various physiological features may manifest in medical images.

The present invention accordingly has a number of advantages. For example, the use of simulated medical imaging systems reduces the need for real medical imaging systems' availability and thus frees up more time for the medical imaging systems to be used in real patient diagnostics. Furthermore, training can be performed in a wider variety of locations (e.g., remotely) rather than requiring students to be physically present with a real medical imaging system. Additionally, using simulated patients reduces the need for actual patients and actual patient data (as, for example, real patient data can be merged into aggregate example patients or the patient data can be entirely synthesized, e.g., generated using a machine-learning model or neural network according to methods the skilled person would be aware of) during training as well as increasing the variety of clinical circumstances which students can have experience with.

Referring now to Fig. 2, there is depicted a flow diagram of a computer-implemented method 200 for simulating an output of a medical imaging system including receiving a control signal according to a proposed embodiment. Steps 110, 120, and 130 are substantially the same the corresponding steps described in relation to method 100 of Fig. 1.

Step 210 comprises receiving a control signal comprising a control parameter of the simulated medical imaging system. A control parameter of the simulated medical imaging system should be understood as any parameter which can affect the output of the simulated medical imaging system for example, imaging settings or the type of / position of anatomy to-be-imaged. For example, in this embodiment, the control signal comprises two control parameters: the first, at least one imaging setting of the simulated medical imaging system; and the other, a selection of anatomy of the anatomical model. An imaging setting of the simulated medical imaging system is to be understood as a defined configuration of an acquisition parameter, a system geometry, and/or a physical modeling option that can determine how image data is generated (simulated), including but not limited to, for example, scanner type, pulse sequence, detector characteristics, reconstruction algorithm, and tissue interaction models etc. A selection of anatomy of the anatomical model is to be understood as for example, selection of part or whole of the anatomical model to simulate the imaging of using the simulated medical imaging system - for example, the chest of the anatomical model may be selected so that the anatomical model is positioned in such a way inside the simulated medical imaging system such that the generated synthetic medical image (in step 130) will be of the simulated patient's chest. In some embodiments, parts of the anatomical model not selected may, for example, be ignored for the purposes of generating the synthetic medical image. This can improve the efficiency of the simulation without reducing its efficacy.

Furthermore, in this embodiment, the control signal further comprises positioning information indicating a first position of the simulated patient in the simulated medical imaging system. As discussed, the position of the simulated patient in the simulated medical imaging system can affect the generation of the synthetic medical image, e.g., what is included in the synthetic medical image. The first position may be an initial position of the simulated patient (i.e., prior to simulated movement of the simulated patient) and/or it may be a desired position of the simulated patient (e.g., set by a user or by the system (e.g., according to the selected anatomy)) which may or may not be adhered to by the simulated patient (e.g., due to simulated behavior/motion of the simulated patient).

Step 220 is thus substantially the same as step 120 described in relation to method 100, however, generating the synthetic medical image is now further based on (i.e., responsive to) the control signal received in step 210.

In step 240, the display control signal is output to a virtual or augmented reality headset such that it displays the synthetic medical image generated in step 220. As the skilled person would know, a virtual or augmented reality headset is a head-mounted display device configured to present imagery, either fully immersive (virtual reality) or overlaid onto the real-world environment (augmented reality), typically incorporating stereoscopic displays, head-tracking sensors, and user interaction interfaces to enable spatially coherent visualization and manipulation of digital content in three dimensions. In other embodiments, the display control signal can be output to any other suitable display device, e.g., a monitor. Advantageously, in this embodiment, the control signal in step 210 is received by the same virtual or augmented reality headset which the display control signal will be output to. This allows a user to directly interact with the simulation, thus facilitating a more intuitive and lifelike experience, e.g., being able to easily adjust control parameters of the simulated medical imaging system. This may be particularly useful for learning purposes when the display control signal also describes a representation of the simulated medical imaging system such that a user can adjust control parameters of the simulated medical imaging system in a lifelike way by directly interacting with the simulated medical imaging system and/or the simulated patient.

In a specific example of the invention, using advanced simulation software and virtual reality (VR) technologies, a highly realistic medical imaging system (e.g., an MRI scanner) interface can be created. A 3D environment can be generated which replicates an actual medical imaging system's controls and displays, thus facilitating trainees having authentic hands-on experience without needing physical access to a real medical imaging system. Intuitive user interfaces can be provided which allow trainees to navigate and interact with the simulation including, for example, adjustable scan parameters, patient table controls, and real-time imaging process management. Haptic feedback may also be integrated to simulate tactile interactions with the simulated equipment, thus enhancing the realism of the experience by, for example, providing physical feedback when uses interact with virtual machine components.

In some specific examples of the invention, a user may provide input as to their specific needs, objectives and prior knowledge such that training modules can be created and customized for the user (e.g., including a variety of example patients and/or imaging settings). Furthermore, machine learning algorithms may dynamically adjust training content based on a user's performance, learning style, and/or progress, thus facilitating each trainee receiving a personalized experience which align with their strengths and areas for improvement. Where there are multiple users, cohort analysis may be conducted to compare trainee performance within groups to, for example, identify common areas for improvement and to help inform the optimization of training strategies. Furthermore, software may be implemented that continuously monitors user/trainee actions during simulation exercises to track interactions, decision-making processes and adherence to clinical procedures. Generative AI may also be integrated to create personalized training content, including instructional videos, interactive guides and best practice recommendations which can, for example, adapt to the trainee's performance in real-time. Additionally, direct instructional content and guidance can be provided to users during training sessions, allowing users to make informed decisions and have their errors corrected immediately therefore reinforcing proper techniques and protocols.

In another specific example of the invention, a secure, centralized database can be established to capture and store all training-related data while using robust encryption protocols to ensure the confidentiality and integrity of training results, protecting sensitive information from unauthorized access.

In yet another specific example of the invention, authentication systems can be used to, for example, implement biometric authentication such as fingerprint scanning or facial recognition to ensure secure access to the training platform, thus safeguarding training data and restricting access to authorized users only.

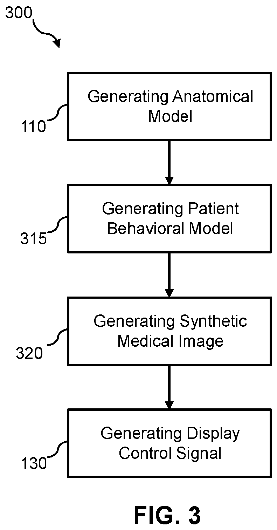

Referring now to Fig. 3, there is depicted a flow diagram of a computer-implemented method 300 for simulating an output of a medical imaging system including generating a patient behavioral model according to a proposed embodiment. Steps 110 and 130 are substantially the same as have been described in relation to the method 100 in Fig. 1.

Step 315 comprises generating a patient behavioral model describing a position and movement of the simulated patient in the simulated medical imaging system. In other words, a patient behavioral model is generated which describes a patient's physical behavior with regards to their position and movement relative to the simulated medical imaging system (i.e., the patient behavioural model indicates a time-dependent position of the patient as a whole and/or positions of various anatomical structures of the patient, e.g., each limb and torso and head, etc.). For instance, it may be useful to simulate a patient breathing and not keeping still such that a user can see how this affects medical images being output from a medical imaging system. Furthermore, in some embodiments, the user may be able to instruct the simulated patient to keep still and/or position/reposition them (e.g., via providing positioning information in a control signal, as described in relation to method 200). In other words, common challenges faced during real-life medical imaging sessions can be mimicked such as patient movement and/or breathing, and using one or more control signals, a user may be able to interact with the simulated patient to, for example, tell the simulated patient to stop moving. In some embodiments, speech synthesis can be used to simulate a patient's speech and speech recognition can be used to allow the user to speak to the simulated patient.

Step 320 is substantially the same as step 120 described in relation to method 100, however, generating the synthetic medical image is now further based on (i.e., responsive to) the patient behavioral model generated in step 315 as this affects the patient's position in the medical imaging system at the time of simulated-imaging and thus naturally affects the synthesized medical image.

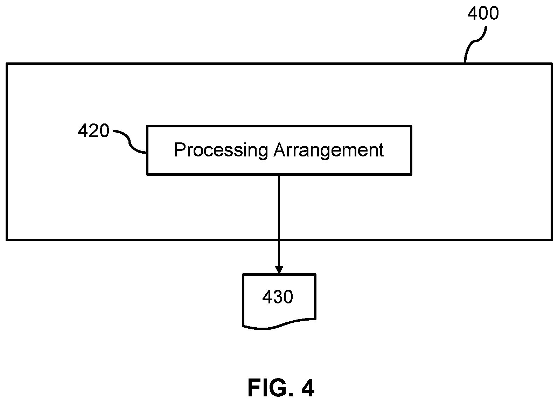

Referring now to Fig. 4, there is depicted a system 400 for simulating an output of a medical imaging system according to a proposed embodiment. The system 400 comprises a processing arrangement 420.

The processing arrangement 420 can be configured to perform any herein-disclosed method (e.g., any of methods 100, 200 or 300). However, in this embodiment, the processing arrangement 420 is specifically configured to perform method 100, i.e., to: generate an anatomical model of a simulated patient based on patient information describing physiological features of an example patient; generate a synthetic medical image of a simulated medical imaging system based on the anatomical model; and generate a display control signal describing the synthetic medical image. The output 430 of the system is thus a display control signal describing the synthetic medical image. In some embodiments, the system 400 may further comprise an input interface configured to, for example, receive a control signal (as described in relation to method 200 of Fig. 2).

In a more specific example of the invention, the main elements of a system according to the invention can be summarized as follows: a patient database containing data describing clinical cases and patient characteristics from which a case for simulation can be selected; an anatomical and pathological model creation module for generating a three-dimensional tissue map according to the selected clinical case including pathological findings and patient-specific features, such as implants; a synthetic image generation module for creating synthetic medical images from the anatomical and pathological model and provided imaging settings as well as from positioning and motion data from a virtual patient model - the image generation can be based on, for example, a Bloch simulator, an AI image generator, a selection from pre-defined images, etc.; a patient conditional and behavioral model creation module for generating a description of the patient's appearance, behavioral patterns, capabilities, impairments, etc. according to the selected patient details; a behavioral state simulation module for tracking the virtual patient's state, generating audio and video data of the virtual patient and changing the state according to a user's interaction with the virtual patient as well as in response to the synthetic image generation module (e.g., simulating rising anxiety in the virtual patient because of simulated MR noise); a simulated imaging system user interface for allowing a user to interact with the synthetic image generation module; and a training augmentation user interface for providing audio and video data of the virtual patient to a user and for allowing a user to communicate with the virtual patient via audio or text, for example.

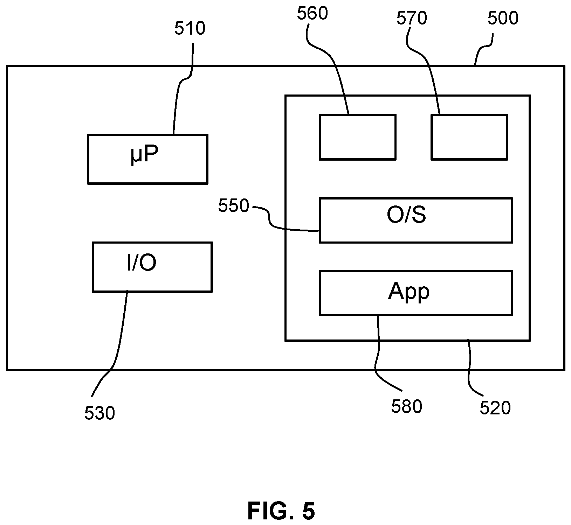

Fig. 5 illustrates an example of a computer 500 within which one or more parts of an embodiment may be employed. Various operations discussed above may utilize the capabilities of the computer 500. In this regard, it is to be understood that system functional blocks can run on a single computer or may be distributed over several computers and locations (e.g. connected via internet).

The computer 500 includes, but is not limited to, PCs, workstations, laptops, PDAs, palm devices, servers, storages, and the like. Generally, in terms of hardware architecture, the computer 500 may include one or more processors 510, memory 520 and one or more I/O devices 530 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 510 is a hardware device for executing software that can be stored in the memory 520. The processor 510 can be virtually any custom made or commercially available processor, a central processing unit (CPU), a digital signal processor (DSP), or an auxiliary processor among several processors associated with the computer 500, and the processor 510 may be a semiconductor-based microprocessor (in the form of a microchip) or a microprocessor.

The memory 520 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 520 may incorporate electronic, magnetic, optical, and/or other types of storage media. Note that the memory 520 can have a distributed architecture, where various components are situated remote from one another, but can be accessed by the processor 510.

The software in the memory 520 may include one or more separate programs, each of which comprises an ordered listing of executable instructions for implementing logical functions. The software in the memory 520 includes a suitable operating system (O/S) 550, compiler 560, source code 570, and one or more applications 580 in accordance with exemplary embodiments. As illustrated, the application 580 comprises numerous functional components for implementing the features and operations of the exemplary embodiments. The application 580 of the computer 500 may represent various applications, computational units, logic, functional units, processes, operations, virtual entities, and/or modules in accordance with exemplary embodiments, but the application 580 is not meant to be a limitation.

The operating system 550 controls the execution of other computer programs, and provides scheduling, input-output control, file and data management, memory management, and communication control and related services. It is contemplated by the inventors that the application 580 for implementing exemplary embodiments may be applicable on all commercially available operating systems.

Application 580 may be a source program, executable program (object code), script, or any other entity comprising a set of instructions to be performed. When a source program, then the program is usually translated via a compiler (such as the compiler 560), assembler, interpreter, or the like, which may or may not be included within the memory 520, so as to operate properly in connection with the O/S 550. Furthermore, the application 580 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 530 may include input devices such as, for example but not limited to, a mouse, keyboard, scanner, microphone, camera, etc. Furthermore, the I/O devices 530 may also include output devices, for example but not limited to a printer, display, etc. Finally, the I/O devices 530 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 530 also include components for communicating over various networks, such as the Internet or intranet.

If the computer 500 is a PC, workstation, intelligent device or the like, the software in the memory 520 may further include a basic input output system (BIOS) (omitted for simplicity). The BIOS is a set of essential software routines that initialize and test hardware at start-up, start the O/S 550, and support the transfer of data among the hardware devices. The BIOS is stored in some type of read-only-memory, such as ROM, PROM, EPROM, EEPROM or the like, so that the BIOS can be executed when the computer 500 is activated.

When the computer 500 is in operation, the processor 510 is configured to execute software stored within the memory 520, to communicate data to and from the memory 520, and to generally control operations of the computer 500 pursuant to the software. The application 580 and the O/S 550 are read, in whole or in part, by the processor 510, perhaps buffered within the processor 510, and then executed.

When the application 580 is implemented in software it should be noted that the application 580 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 580 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1-3, and the system of Fig. 4, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 5 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

## Claims

1. A computer-implemented method (100) for simulating an output of a medical imaging system, the method comprising:
generating an anatomical model (110) of a simulated patient based on patient information describing physiological features of an example patient;
generating a synthetic medical image (120) of a simulated medical imaging system based on the anatomical model; and
generating a display control signal (130) describing the synthetic medical image.

2. The computer-implemented method of claim 1, further comprising receiving a control signal (210) comprising a control parameter of the simulated medical imaging system, wherein the synthetic medical image is generated further based on the control signal.

3. The computer-implemented method of claim 2, wherein the control parameter comprises a selection of anatomy of the anatomical model.

4. The computer-implemented method of claim 2 or 3, wherein the control parameter comprises at least one imaging setting of the simulated medical imaging system.

5. The computer-implemented method of any of claims 2 to 4, wherein the control signal further comprises positioning information indicating a first position of the simulated patient in the simulated medical imaging system.

6. The computer-implemented method of any preceding claim, wherein generating the synthetic medical image comprises using a Bloch simulation algorithm.

7. The computer-implemented method of any preceding claim, wherein the patient information comprises a patient-specific feature, and optionally, wherein the patient-specific feature comprises at least one of: an implant; a bone fracture; a soft tissue injury; a tumor; a surgical modification; scar tissue; inflammation; an anatomical abnormality; and a foreign body.

8. The computer-implemented method of any preceding claim, further comprising:
generating a patient behavioral model (315) describing a position and movement of the simulated patient in the simulated medical imaging system, wherein the synthetic medical image is generated further based on the patient behavioral model.

9. The computer-implemented method of any preceding claim, wherein the anatomical model comprises a 3D tissue map.

10. The computer-implemented method of any preceding claim, wherein the display control signal further describes a representation of the simulated medical imaging system.

11. The computer-implemented method of any preceding claim, wherein the method further comprises outputting the display control signal to a virtual or augmented reality headset.

12. The computer-implemented method of claim 11, dependent on any of claims 2 to 5, wherein the control signal is received by the virtual or augmented reality headset.

13. The computer-implemented method of any preceding claim, wherein the medical imaging system comprises one of: an MRI system; a CT system; an X-ray system; and an ultrasound system.

14. A computer program product comprising computer program code means which, when executed on a computing device having a processing system, cause the processing system to perform all of the steps of the computer-implemented method according to any of claims 1 to 13.

15. A system (500) for simulating an output of a medical imaging system, the system comprising:
a processing arrangement (520) configured to:
generate an anatomical model of a simulated patient based on patient information describing physiological features of an example patient;
generate a synthetic medical image of a simulated medical imaging system based on the anatomical model; and
generate a display control signal describing the synthetic medical image.
